# EUROPEAN PATENT APPLICATION

(11) **EP 3 031 398 A1**
(43) Date of publication of application: **15.06.2016**
(21) Application number: 15174056.0
(22) Date of filing: 26.06.2015
(51) Int. Cl.: A61B 8/00

(54) **ULTRASOUND DIAGNOSTIC APPARATUS AND CONTROL METHOD FOR THE SAME**

(30) Priority: 08.12.2014 KR 20140175145
(71) Applicant: Samsung Medison Co., Ltd., Gangwon-do 250-870 (KR)
(72) Inventor: Lee, Jun Kum, Seoul (KR); Kim, Jung, Seoul (KR); Kang, Yeon Ah, Seoul (KR); Kim, Deok Gon, Seoul (KR); Hwang, Yoon Gu, Gyeonggi-do (KR)
(74) Representative: Frey, Sven Holger

(57) **Abstract**

Disclosed herein are an ultrasound diagnostic apparatus and a control method for the same, and more particularly, an apparatus to audio-visually inform information on the actions needed to be taken by a patient at the time of an ultrasound diagnosis. In accordance with one aspect of the present disclosure, an ultrasound diagnostic apparatus includes a storage unit at which data of actions of a patient needed to be taken at the time of an ultrasound diagnosis is stored; a processor to determine a type of an ultrasound probe or a diagnostic mode provided to perform an ultrasound diagnosis and select from the data of actions stored as the above the data of action to be taken by the patient on the basis of the type of the ultrasound probe or the diagnostic mode determined as the above; and at least one display unit to display the selected data of action to be taken by the patient.

## Description

### BACKGROUND

### 1. Field

Embodiments of the present disclosure relate to an ultrasound diagnostic apparatus and a control method for the same, and more particularly, an apparatus to audio-visually inform information on the actions needed to be taken by a patient at the time of an ultrasound diagnosis.

### 2. Description of the Related Art

An ultrasound diagnostic apparatus is provided to be used for medical purposes such as observations of an inside a subject, detections of foreign subjects, and measurements of bodily harm by radiating ultrasound signals generated from a transducer of a probe from a surface of a body of the subject toward a portion of a target at an inside the body and by noninvasively obtaining images with respect to cross sections or blood flow of a soft tissue of the portion at the inside the subject by receiving information about the reflected ultrasound signals, that is, the ultrasound echo signals.

The ultrasound diagnostic apparatus as such, when compared to other image diagnostic apparatuses such as an x-ray diagnostic apparatus, an x-ray CT scanner (Computerized Tomography Scanner), an MRI (Magnetic Resonance Image), and a nuclear medicine diagnostic apparatus, is provided in small size and less expensive, while capable of displaying in real time and safer due to less exposure of radiation, and thus is widely used along with other image diagnostic apparatuses.

In a case of diagnosing by use of the ultrasound diagnostic apparatus, the actions to be taken by a patient are varied depending on the subject portions of diagnosis.

### SUMMARY

Therefore, it is an aspect of the present disclosure to provide an ultrasound diagnostic apparatus configured to inform information on actions needed to be taken by a patient at the time of an ultrasound diagnosis and a method for the same, and by audio-visually providing the information on the various actions according to the portions to be scanned, convenience and usefulness in ultrasound diagnosis are to be enhanced.

Additional aspects of the disclosure will be set forth in part in the description which follows and, in part, will be obvious from the description, or may be learned by practice of the disclosure.

In accordance with one aspect of the present disclosure, an ultrasound diagnostic apparatus includes a storage unit, a processor and at least one display unit. The storage unit may store data of actions of a patient needed to be taken at the time of an ultrasound diagnosis. The processor may determine a type of an ultrasound probe or a diagnostic mode provided to perform an ultrasound diagnosis and select a data of action to be taken by the patient from the stored data of actions on the basis of the determined type of the ultrasound probe or the determined diagnostic mode. The at least one display unit may display the selected data of action to be taken by the patient.

The at least one display unit may include a first display unit and a second display unit.

The ultrasound diagnostic apparatus may include a first alarm unit to inform the selected data of action to be taken by the patient by use of an speech signal.

The second display unit may include a second alarm unit to inform the selected data of action to be taken by the patient by use of an speech signal.

The ultrasound diagnostic apparatus may include a communication unit to transmit the selected data of actions to be taken by the patient to the second display unit and the second alarm unit.

The ultrasound diagnostic apparatus may include an input unit to receive an input of the data of actions to be taken by the patient.

The input unit may receive an input as to change or reset the selected data of actions to be taken by the patient.

The ultrasound diagnostic apparatus may further include a probe-type determining unit to determine the type of an ultrasound probe to perform the ultrasound diagnosis by recognizing the type of a male connector coupled to a female connector.

The first display unit and the second display unit may simultaneously display an ultrasound diagnostic image and the selected data of actions to be taken by the patient.

In accordance with another aspect of the present disclosure, an ultrasound diagnostic method includes: determining a type of an ultrasound probe or a diagnostic mode provided to perform an ultrasound diagnosis; selecting a data of action to be taken by a patient from stored data of actions on the basis of the determined type of the ultrasound probe or the determined diagnostic mode; and displaying the selected data of action to be taken by the patient.

The ultrasound diagnostic method may further include informing the selected data of action to be taken by the patient by use of an speech signal.

The ultrasound diagnostic method may further include transmitting the selected data of actions to be taken by the patient when the data of actions are selected.

The ultrasound diagnostic method may include receiving an input of the data of actions to be taken by the patient.

The receiving of the input of the data of actions to be taken by the patient may include receiving an input as to change or reset the selected data of actions to be taken by the patient.

The determining of the type of an ultrasound probe may include determining the type of the ultrasound probe to perform the ultrasound diagnosis by recognizing the type of a male connector coupled to a female connector.

The displaying of the selected data of actions to be taken by the patient may include simultaneously displaying an ultrasound diagnostic image and the selected data of actions to be taken by the patient.

### BRIEF DESCRIPTION OF THE DRAWINGS

These and/or other aspects of the disclosure will become apparent and more readily appreciated from the following description of the embodiments, taken in conjunction with the accompanying drawings of which:
FIG. 1 is a perspective view illustrating an ultrasound diagnostic apparatus in accordance with one embodiment of the present disclosure.
FIG. 2 is a drawing illustrating actions to be taken by a patient at the time of when performing an ultrasound diagnosis with respect to a heart in accordance with one embodiment of the present disclosure.
FIG. 3 is a drawing illustrating actions to be taken by a patient at the time of when performing an ultrasound diagnosis with respect to an abdomen in accordance with one embodiment of the present disclosure.
FIG. 4 is a drawing illustrating actions to be taken by a patient at the time of when performing an ultrasound diagnosis with respect to a carotid in accordance with one embodiment of the present disclosure.
FIG. 5 is a drawing showing differences of the types of ultrasound probes according to diagnostic portions.
FIG. 6 is a controlled block diagram of the ultrasound diagnostic apparatus in accordance with one embodiment of the present disclosure.
FIG. 7 is a flow chart illustrating a control method of the ultrasound diagnostic apparatus provided with a processor as to select data of actions to be taken by a patient in accordance with one embodiment of the present disclosure.
FIG. 8 is a flow chart illustrating a method of controlling the ultrasound diagnostic apparatus provided as to input the data of actions to be taken by a patient through an input unit in accordance with one embodiment of the present disclosure.
FIG. 9 is a drawing illustrating a second display unit at which the data of actions to be taken by a patient is displayed at the time of performing an ultrasound diagnosis on an abdomen in accordance with one embodiment of the present disclosure.
FIG. 10 is a drawing illustrating the second display unit at which the data of actions to be taken by a patient is displayed at the time of performing an ultrasound diagnosis on a carotid in accordance with one embodiment of the present disclosure.
FIG. 11 is a drawing illustrating the second display unit at which the data of actions to be taken by a patient is displayed at the time of performing an ultrasound diagnosis on a heart in accordance with one embodiment of the present disclosure.
FIG. 12 is a perspective view illustrating the ultrasound diagnostic apparatus having a first display unit into which an ultrasound diagnostic apparatus for an abdomen is coupled at the time of an ultrasound diagnosis of an abdomen and at which the data of actions to be taken by a patient is displayed in accordance with one embodiment of the present disclosure.
FIG. 13 is a perspective view illustrating the ultrasound diagnostic apparatus having the first display unit into which an ultrasound diagnostic apparatus for a heart is coupled at the time of an ultrasound diagnosis of a heart and at which the data of actions to be taken by a patient is displayed in accordance with one embodiment of the present disclosure.

### DETAILED DESCRIPTION

Reference will now be made in detail to the embodiments of the present disclosure, examples of which are illustrated in the accompanying drawings, wherein like reference numerals refer to like elements throughout.

As for a diagnostic apparatus which may be applied with technologies with respect to an ultrasound diagnostic apparatus and a control method for the same in accordance with one embodiment of the present disclosure may be referred to as certain one of an x-ray photographing apparatus, an x-ray radiographic apparatus, a CT scanner, a MRI, positron emission tomography apparatus, and an ultrasound diagnostic apparatus, and in the descriptions with respect to the present embodiments, case of the ultrasound diagnostic apparatus will be used as an example while the such is not limited hereto.

In addition, terminologies such as 'a user' and 'a test operator' used hereinafter are referred to as medical professions including doctors, nurses, clinical technologists, medical imaging technologists, and sonographers, or even repair technicians of medical apparatuses, but are not limited hereto.

The present disclosure is related to an ultrasound diagnostic apparatus and a control method for the same, and conventionally, in a case of performing an ultrasound diagnosis of a heart, an abdomen, or carotid, a patient is verbally informed by the test operator as to take actions that are proper for diagnosis of performing the diagnosis. In the case as such, the patient may not be able to take the proper actions needed for the diagnosis while not being able to clearly recognize the actions for the diagnosis, and thus the convenience of an ultrasound diagnosis is reduced and time consumption is increased. The present disclosure is suggested as to solve the difficulties as such, and as a patient is able to visually observe the proper actions needed to be taken for an ultrasound diagnosis and take the appropriate actions accordingly, the convenience of an ultrasound diagnosis is increased and furthermore, by providing the actions to be taken through speech signals, the test operator is not needed to repeatedly inform the patient with respect to the actions to be taken.

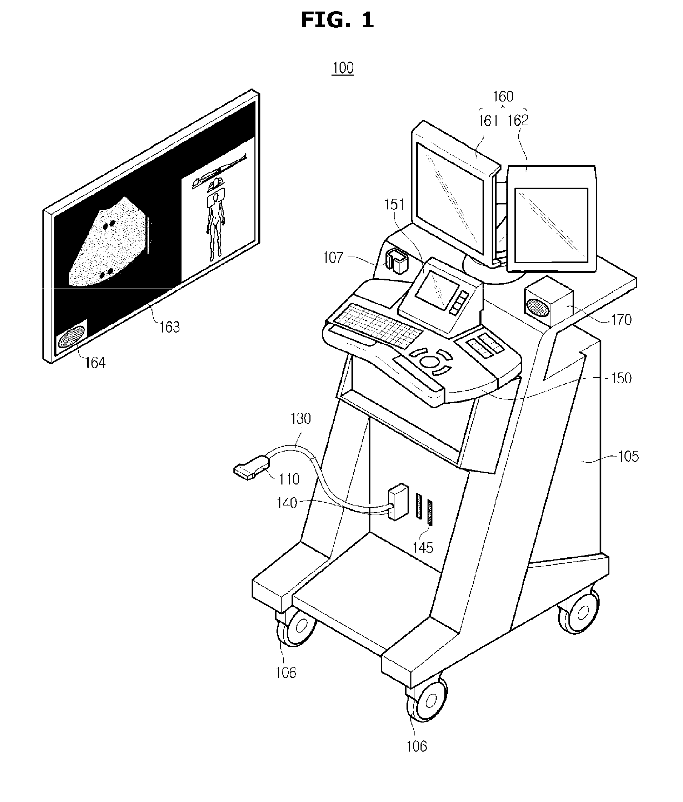

FIG. 1 is a perspective view illustrating an ultrasound diagnostic apparatus in accordance with one embodiment of the present disclosure.

As illustrated on FIG. 1, an ultrasound diagnostic apparatus 100 may include a probe 110, a body 105, an input unit 150, a first display unit 160, a second display unit 163, a first alarm unit 170, and a second alarm unit 164.

At least one female connector 145 may be provided at a front of a lower side of the body 105. A male connector 140 provided at one end of a cable 130 may be physically coupled to the female connector 145. The ultrasound probe 110 and the body 105 may be connected to each other through the cable 130.

Meanwhile, a plurality of casters 106 provided for movability of the ultrasound diagnostic apparatus may be provided at a lower portion of the body 105. A user may be able to fix the ultrasound diagnostic apparatus 100 at a certain position by use of the plurality of casters 106, or may move the ultrasound diagnostic apparatus 100 toward a certain direction. The ultrasound diagnostic apparatus 100 as such is referred to as a cart-type ultrasound diagnostic apparatus.

Meanwhile, differently from FIG. 1, the ultrasound diagnostic apparatus 100 may be referred to as a portable ultrasound diagnostic apparatus configured to be carried along at the time of moving a long distance. At this time, the portable ultrasound diagnostic apparatus may not be provided with casters. As for examples of the portable ultrasound diagnostic apparatus 100, a PACS viewer, a smart phone, a lap-top computer, a PDA, and a tablet PC may be present, but are not limited hereto.

The ultrasound probe 110 is referred to as a portion making contact with respect to a surface of a body of a subject, and is capable of transmitting/receiving ultrasound waves to the subject. In detail, the ultrasound probe 110 is provided to generate ultrasound waves according to the pulse being input and to transmit the ultrasound waves to an inside the subject, and is provided to receive the echo ultrasound waves that are reflected from a particular portion at the inside the subject.

As the actions of a patient needed to be taken at the time of an ultrasound diagnosis vary, the types of the ultrasound probe 110 may vary according to the portions of ultrasound diagnosis, and depending on the type of the ultrasound probe, the position of a connector being mounted may vary as well.

The input unit 150 is referred to as a portion capable of receiving commands with respect to motions of the ultrasound diagnostic apparatus 100. A user may be able to input commands to perform a starting of diagnosis, a selection of portion of diagnosis, a selection of type of diagnosis, a selection of modes related to ultrasound images being finally output. As for the modes related to the ultrasound images, an A-mode (Amplitude mode), a B-mode (Brightness mode), a D-mode (Doppler mode), an E-mode (Elastography mode), and a M-mode (Motion mode) may be presented as examples.

As illustrated on FIG. 1, as one example, the input unit 150 may be positioned at an upper portion of the body 105. At this time, the input unit 150 may include one of a switch, a key, a wheel, a joystick, a track ball, and a knop.

In addition, a sub display 151 is capable of displaying menu or information needed in setting the ultrasound diagnostic apparatus 100 or displaying th settings of the ultrasound diagnostic apparatus 100 at present.

At this time, the sub display 151 may be implemented in the form of a touch panel, and in a case when the sub display 151 is implemented in the form of the touch pad, a user may be able to input control commands by touching the sub display 151.

The sub display 151, for example, may be implemented in the form of a Liquid Crystal Display (LCD) panel, a Light Emitting Diode (LED) panel, or an Organic Light Emitting Diode: OLED) panel.

At least one probe holder 107 configured to dispense the ultrasound probe 110 may be provided at the surroundings of the input unit 150. Thus, a user, when not using the ultrasound diagnostic apparatus 100, may be able to dispense and store the ultrasound probe 110 at the probe holder 107.

The first display unit 160 may be able to display ultrasound images obtained during an ultrasound diagnostic process. In addition, the data of actions to be taken by a patient in accordance with one embodiment of the present disclosure may be displayed. As shown on FIG. 1, the first display unit 160 may be mounted at the body 105 while coupled to the body 105, or may be implemented as to be detached from the body 105.

In addition, the first display unit 160 is provided with a plurality of display apparatuses 161 and 162, and may be able to simultaneously display different images. For example, the first display apparatus 161 may be able to display ultrasound images obtained by photographing a subject, and the second display apparatus 162 may be able to display matching images. The first display apparatus 161 may be able to display 2D images obtained by photographing a subject, and the second display apparatus 162 may be able to display 3D images. In addition, the first display apparatus 161 may be able to display ultrasound images obtained during an ultrasound diagnostic process, and the second display apparatus 162 may be able to display images with respect to the actions to be taken by a patient. The ultrasound diagnostic images and the images with respect to the actions to be taken by a patient as a whole may be displayed at the first display apparatus 161.

In addition, the each of the display apparatuses 161 and 162 may employ means of display, such as a Plasma Display Panel (PDP), a Liquid Crystal Display (LCD) panel, a Light Emitting Diode (LED) panel or an Organic Light Emitting Diode (OLED) panel, or an Active-matrix Organic Light-Emitting Diode (AMOLED) panel.

The first alarm unit 170 may be mounted at a random position of the body 105. The first alarm unit may be able to output the data of actions to be taken by a patient at the time of an ultrasound diagnosis in the form of speech signal so that the patient or a test operator may be able to recognize, and the speech signal may be provided in the form of human voice to describe the actions to be taken by a patient in detail, or may be provided in the form of mechanical sound as to have a patient recognize the data of actions or the data of actions displayed on a screen. By using an example of the case of the signal in the form of human voice describing the actions to be taken by a patient in detail, the signal may be output with the contents such as, "Please lie down on the bed on your left side while your right arm is placed on your right side of the body, the left arm is stretched above your head, and your legs are in bent position," and in a case of the mechanical sound, when the actions to be taken by a patient is displayed on a screen, a mechanical sound, such as "Beep," may be output so that the patient may be able to recognize the actions to be taken by referring to the screen. The first alarm unit 170 may be provided in the form of a general speaker, or may be provided in the form of a diaphragm to output sound only. The position of the installation of the first alarm unit 170 is not limited, and may be installed at a random position of the ultrasound diagnostic apparatus, and thus the body 105 may be installed at one side surface of the input unit 150.

The second display unit 163 may be able to display ultrasound images obtained during an ultrasound diagnostic process as in the case of the first display unit 160. In addition, the second display unit 163 may be able to display the data of actions to be taken by a patient in accordance with one embodiment of the present disclosure. As illustrated on FIG. 1, the second display unit 163 may be separately installed from the body 105 of the ultrasound diagnostic apparatus, and may be installed at a position from which a patient may be able to check a screen while in a state of lying down on a bed or a diagnostic table for an ultrasound diagnosis. The second display unit 163, through a communication unit 220, may be able to receive the data of actions of a patient from the ultrasound diagnostic apparatus for an output, and may also be able to receive the data through a wire without going through the communication unit 220. The second display unit 163 may be able to display ultrasound images obtained during an ultrasound diagnostic process, and along with the ultrasound images obtained during the ultrasound diagnostic process, the second display unit 163 may also be able to display the images with respect to the actions to be taken by a patent. The second display unit 163 may be able to display the second alarm unit 164. As illustrated on FIG. 1, the second alarm unit 164 may be positioned at one side surface of the second display unit 163, and may be of an alarm unit in the form of a speaker that may be recognized from an outside, or may be an alarm unit in the form of a speaker provided at the second display unit 163. The second alarm unit 164, away from the second display unit 163, may be able to receive the data of actions of a patient from the ultrasound diagnostic apparatus for an output, and may also output an alarm by use of the data that is received from the second display unit 163.

The second alarm unit 164 may be able to output the data of actions to be taken by a patient at the time of an ultrasound diagnosis in the form of speech signal, so that the patient may be able to recognize such, and the speech signal may be of signal in the form of human voice to describe the actions to be taken by the patient in detail, or may be of mechanical sound as to simply have the patient recognize with respect to the data of actions or the data of actions displayed on a screen. By using an example of the case of the signal in the form of human voice describing the actions to be taken by a patient in detail, the signal may be output with the contents such as, "Please lie down on the bed on your left side while your right arm is placed on your right side of the body, the left arm is stretched above your head, and your legs are in bent position," and in the case of the mechanical sound, when the actions to be taken by a patient is displayed on a screen, a mechanical sound, such as "Beep," may be output so that the patient may be able to recognize the actions to be taken by referring to the screen.

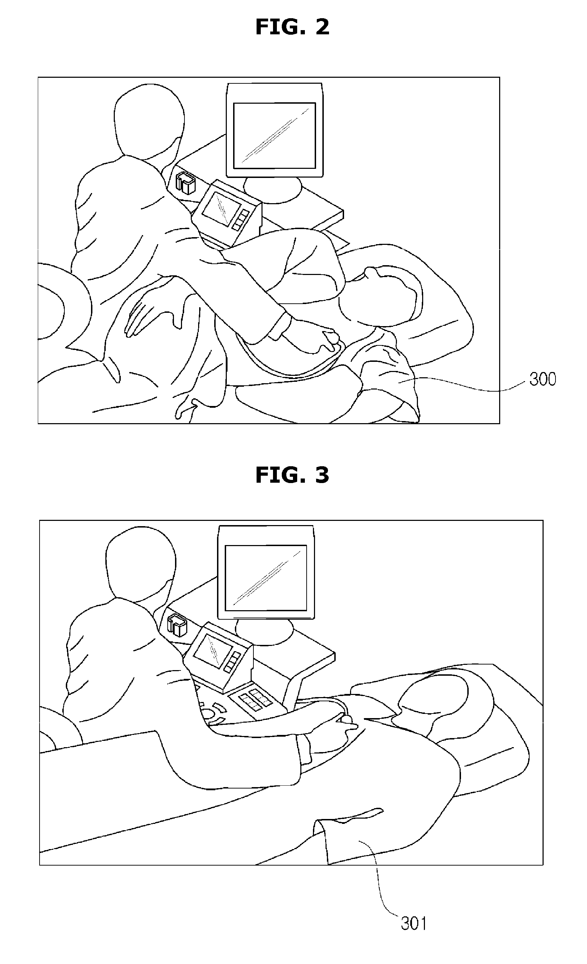

FIG. 2 is a drawing illustrating actions to be taken by a patient at the time of when performing an ultrasound diagnosis with respect to a heart in accordance with one embodiment of the present disclosure.

As described above, in a case of an ultrasound diagnosis, a patient is needed to take various actions according to the portions of the diagnosis, and in a case of taking proper actions for the diagnosis, the ultrasound diagnosis may be precisely performed. As illustrated on FIG, 2, the actions (300) that a patient is needed to take in a case when an ultrasound diagnosis is performed with respect to a heart are to lie down in a reclined manner toward left side direction while the right arm is placed on the right side of the body or comfortably positioned, as the left arm is stretched toward an upper side while legs are bent. When the patient is taken with the actions as such, a test operator may be able to scan the heart at the left portion of the chest of the patient by use of an ultrasound probe for a heart 111. In a case when the patient is not properly taken with the actions described above at the time of diagnosing the heart, the diagnosis may not be properly performed, and thus another diagnosis is needed to be performed.

FIG. 3 is a drawing illustrating the actions to be taken by a patient at the time of when performing an ultrasound diagnosis with respect to an abdomen in accordance with one embodiment of the present disclosure.

As illustrated on FIG. 3, the actions (301) that a patient is needed to take in a case when an ultrasound diagnosis is performed with respect to an abdomen are to lie down with the back on a bed or a table while facing a ceiling while the legs are straightened as the left arm and the right arm are placed on the left side and the right side of the body or the left arm and the right arm may be gathered together. When the patient is taken with the actions as such, a test operator may be able to scan the abdomen by use of an ultrasound probe for an abdomen 113. In a case when the patient is not properly taken with the actions described above at the time of diagnosing the abdomen, the diagnosis may not be properly performed, and thus another diagnosis is needed to be performed.

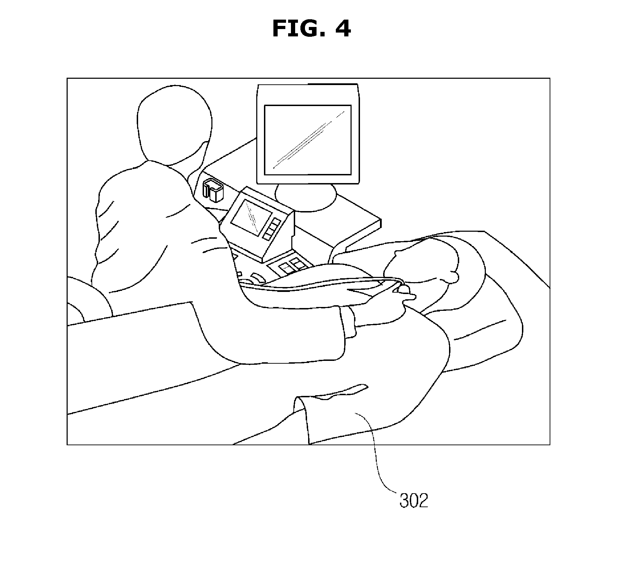

FIG. 4 is a drawing illustrating the actions to be taken by a patient at the time of when performing an ultrasound diagnosis with respect to a carotid in accordance with one embodiment of the present disclosure.

A carotid ultrasound diagnosis is referred to as a test provided as to check the degree of narrowness or position, as well as appropriacy of blood flow of the carotid or the arteria vertebralis positioned at a neck portion by use of ultrasound waves, and as illustrated on FIG, 4, the actions (302) that a patient is needed to take in a case when an ultrasound diagnosis is performed with respect to a carotid are to lie down comfortably with the back on a bed or a table while facing left or right so that the diagnosis with respect to the neck portion may be performed. When the patient is taken with the actions as such, a test operator may be able to scan the carotid by use of an ultrasound probe for a carotid 112. In a case when the patient is not properly taken with the actions described above at the time of diagnosing the carotid, the diagnosis may not be properly performed, and thus another diagnosis is needed to be performed.

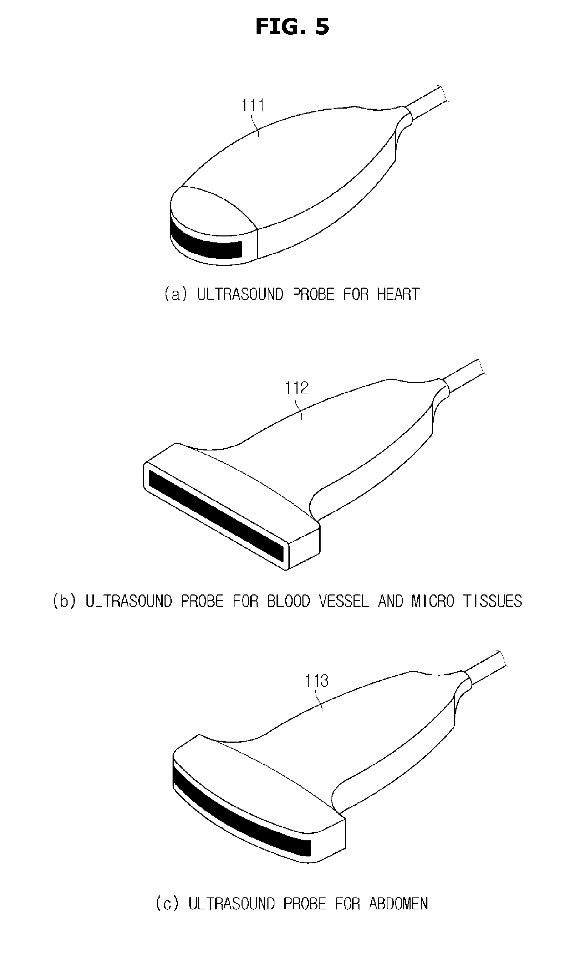

FIG. 5 is a drawing showing differences of the types of ultrasound probes according to diagnostic portions.

As illustrated on FIG. 5, the ultrasound probe may be divided into various types according to the portions or subjects to be diagnosed, and depending on the purpose of test, the size and the shape thereof may be different. An (a) is referred to the ultrasound probe for a heart 111 configured to diagnose a heart in accordance with one embodiment of the present disclosure, a (b) is referred to the ultrasound probe 112 configured to diagnose blood vessel and micro tissues in accordance with one embodiment of the present disclosure, and a (c) is referred to the ultrasound probe for an abdomen 113 configured to diagnose an abdomen in accordance with one embodiment of the present disclosure. On FIG. 5, while the three types of the ultrasound probes are described for the sake of convenience as to describe one embodiment of the present disclosure, the types and the number of the ultrasound probes are not limited, and even in a case of the probes to diagnose a same portion, the shapes and the sizes of the ultrasound probes may be different to each other. That is, the shapes and the sizes of the ultrasound probes may be different according to the portions of diagnosis, and thus, with respect to the ultrasound diagnostic apparatus 100 illustrated on FIG. 1, the ultrasound probe 110 may be replaced with a probe which corresponds to the purpose of ultrasound diagnosis. Depending on the type of the ultrasound probe, the male connector 140 of the probe may be changed, and according to the male connector 140 of the probe, the probe may be coupled to the female connector 145 which corresponds to the male connector 140. As to be described later, on the basis of the purpose of diagnosis or the portion of diagnosis by use of the ultrasound probes having different types with respect to each other, the actions to be taken by a patient by use of the ultrasound diagnostic apparatus and the control method for the same in accordance with one embodiment of the present disclosure are selected, and the selected actions may be displayed on the first display unit and the second display unit.

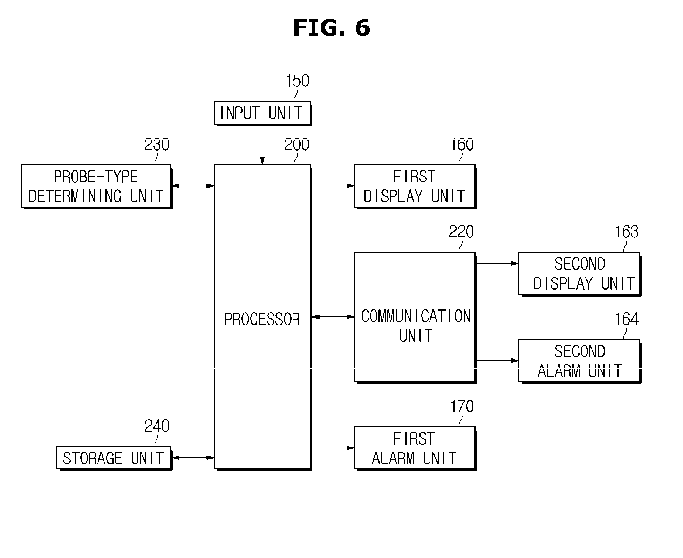

FIG. 6 is a controlled block diagram of the ultrasound diagnostic apparatus in accordance with one embodiment of the present disclosure.

As illustrated on FIG. 6, the ultrasonic diagnostic apparatus 100 includes the first display unit 160, the second display unit 163, the second alarm unit 164, the first alarm unit 170, a processor 200, the input unit 150, the communication unit 220, a probe-type determining unit 230, and a storage unit 240.

The processor 200 is configured to perform a role to control each component of the ultrasound diagnostic apparatus 100, and the each component may be able to perform functions through reciprocal actions with respect to the processor 200. In addition, the processor 200, when the type of the ultrasound probe configured to perform ultrasound diagnosis is determined by use of the probe-type determining unit 230, may be able to select the data of actions to be taken by a patient from the data of actions of the patient that is stored on the basis of the type of the determined ultrasound probe. In detail, the processor 200 may first be able to control the probe-type determining unit 230 as to determine the type of the ultrasound probe provided to perform ultrasound diagnosis. As described on FIG. 5, the type and the size of the ultrasound probe may vary depending on the portion of diagnosis or the purpose of diagnosis, and the actions to be taken by a patient may vary as well. As illustrated on FIG. 1, with respect to the ultrasound diagnostic apparatus 100, the at least one female connector 145 may be provided at a front surface of a lower side of the body 105, and the male connector 140 provided at one end of the cable 130 may be physically coupled unto the female connector 145. Depending on the type of the ultrasound probe, the male connector 140 connected through the cable 130 may vary, and the female connector 145 coupled to the male connector 140 may also vary. In a case when a probe corresponding to the portion of ultrasound diagnosis is coupled, the processor 200, on the basis of the type of the probe determined by use of the probe-type determining unit 230, the data of actions that is proper for diagnosis of a patient may be selected.

In addition, the processor 200 is able to determine ultrasound diagnostic mode, and may be able to select the data of actions to be taken by a patient from the data of actions of the patient that is stored on the basis of the determined ultrasound diagnostic mode. The ultrasound diagnostic mode may include the modes with respect to portions of diagnosis, types of diagnosis, or ultrasound images. The portion of diagnosis, as described on FIG. 2, FIG. 3 and FIG, 4, may correspond to the portions needed for diagnosis with respect to a subject, such as a heart, an abdomen, or a carotid. As the portion of diagnosis as such is determined, on the basis of the determined ultrasound diagnostic portion, the data of actions to be taken by a patient may be selected from the stored data of actions of the patient. The type of ultrasound diagnosis as well may vary according to the portion of diagnosis. With respect to the modes of the ultrasound images, as described on FIG. 1, the A-mode (Amplitude mode), the B-mode (Brightness mode), the D-mode (Doppler mode), the E-mode (Elastography mode), and the M-mode (Motion mode) may be presented as examples.

The processor 200 may be implemented by use of arrays of a plurality of logic gates, or may be implemented by a combination of a general micro processor and a memory at which the programs configured to be executed at the micro processor as such is stored. For example, the processor 200 may be implemented by use of a general GPU.

The storage unit 240 may be able to store the data of actions of a patient based on the portion of ultrasound diagnosis. As described on FIG. 2, FIG. 3 and FIG. 4, the actions to be taken by a patient according to the portions of ultrasound diagnosis vary, and taking the precise actions is needed for precise diagnosis. The actions of a patient according to the portion and purpose of ultrasound diagnosis may be stored in the form of data at the storage unit 240. For example, as illustrated on FIG. 2, regarding the data of actions of a patient with respect to the ultrasound diagnosis of a heart, the posture of lying down in a reclined manner toward left side direction while the right arm is placed on the right side of the body or comfortably positioned, as the left arm is stretched toward an upper side while legs are bent may be stored in the form of data. The processor 200 may be able to select the data of actions to be taken by a patient from the data of actions of the patient stored at the storage unit 240.

The storage unit 240, for example, may include a high-speed random access memory, a magnetic disc, a SRAM, a DRAM, or a ROM, but is not limited hereto. In addition, the storage unit 240 may be detached with respect to the ultrasound diagnostic apparatus 100. For example, the storage unit 240 may include a CF card (Compact Flash Card), a SD card (Secure Digital Card), a SM card (Smart Media Card), a MMC (Multimedia Card), or a memory stick, but is not limited hereto. In addition, the storage unit 240 is provided at an outside the ultrasound diagnostic apparatus 100, and may be able to transmit or receive data with respect to the ultrasound diagnostic apparatus 100 wirelessly or through wire.

A test operator or a user to perform an ultrasound diagnosis may be able to input the data of actions to be taken by a patient through the input unit 150 on the basis of the portion of ultrasound diagnosis. The input unit 150 may include at least one of a switch, a key, a wheel, a joystick, a track ball, and a knop. In addition, a GUI (Graphical User Interface) such as a touch pad, that is, an input apparatus which is software, may be included as to provide an input by a user. The touch pad may be implemented in the form of a Touch Screen Panel (TSP). The test operator or the user may be able to select actions of a patient as to correspond to an ultrasound diagnosis, and by having the selected actions displayed on a screen by inputting the selected actions through the input unit 150, the difficulty of having to verbally inform the patient in a repeated manner with respect to the actions is reduced. In addition, the test operator or the user may be able to perform an input as to change or reset the data of actions to be taken by a patient, which are selected by use of the processor 200 through the input unit 150. In a case when a need to revise or change the data of actions of a patient according to the portions of diagnosis or in a case when an error is present in the selection of the data of the processor 200, the test operator or the user may be able to perform an input to newly input or change the data of the patient.

The communication unit 220 may be able to transmit the data of actions to be taken by a patient, which are selected by use of the processor 200, to the second display unit 163 and the second alarm unit 164. The second display unit 163 may include the second alarm unit 164, and may be connected to the ultrasound diagnostic apparatus 100 wirelessly or through wire. In a case of being connected by use of wire, the data of actions to be taken by a patient may be directly sent to the second display unit 163 and the second alarm unit 164, but in a case of being wirelessly connected, the data may be sent to the second display unit 163 and the second alarm unit 164 through the communication unit 220. The communication unit 220 may communicate data in between the processor 200 and the second display unit 163 and in between the processor 200 and the second alarm unit 164 according to various wire/wireless communication protocols. However, the data communication is desired to be taken place according to the standards of Digital Imaging and Communications in Medicine (DICOM).

The probe-type determining unit 230 is configured to determine for which portion an ultrasound diagnostic probe is used, by recognizing the ultrasound probe 110 connected to the ultrasound diagnostic apparatus 100. The male connector 140 and the female connector 145 corresponding to the male connector 140 are varied depending on the type of an ultrasound probe, and thus a determination may be made for which portion an ultrasound diagnosis is performed by recognizing which type of the ultrasound probe is mounted. The probe-type determining unit 230 may be able to determine the type of a corresponding ultrasound probe on the basis of the pre-stored information with respect to the ultrasound probes according to portions of diagnosis, when the male connector 140 is coupled to the female connector 145.

The first display unit 160 and the second display unit 163 may be able to display data of actions to be taken by a patient, which are selected by use of the processor 200. As described above, the first display unit 160 is generally coupled to and mounted at the body 105, and may include the plurality of display apparatuses 161 and 162. The first display apparatus 161 is provided as to display ultrasound images obtained during an ultrasound diagnostic process, and the second display apparatus 162 is provided as to display imaged wit respect to the actions to be taken by a patient, while the entirety of the ultrasound diagnostic images and the images with respect to the actions to be taken by a patient may be displayed at the first display apparatus 161.

The second display unit 163 may be able to display ultrasound images obtained during an ultrasound diagnostic process as in the case of the first display unit 160, and may simultaneously be able to display the data of actions to be taken by a patient, which are selected by use of the processor 200 as well. The second display unit 163 may be able to receive the data of actions to be taken by a patient by use of wire, or may be able to wirelessly receive the data of actions to be taken by a patient through the communication unit 220.

The first alarm unit 170 and the second alarm unit 164 may be able to output the data of actions to be taken by a patient, which are selected by use of the processor 200, in the form of speech signal so that the patient or a test operator may be able to recognize, and the speech signal may be provided in the form of human voice or mechanical sound. The first alarm unit 170 may be mounted at a random position of the body 105, and the second alarm unit 164 may be included in the second display unit 163. The first alarm unit 170 may be able to output speech signal by receiving the data of actions to be taken by a patient, which are selected by use of the processor 200, and the second alarm unit 164 may be able to output an alarm through the communication unit 200 by receiving the data of actions to be taken by a patient. The first alarm unit 170 may be provided in the form of a general speaker, or may be provided in the form of a diaphragm to output sound only, while the second alarm unit 164, as illustrated on FIG. 1, may be positioned at one side surface of the second display unit 163, and may be of an alarm unit in the form of a speaker that may be recognized from an outside, or may be an alarm unit in the form of a speaker provided at the second display unit 163.

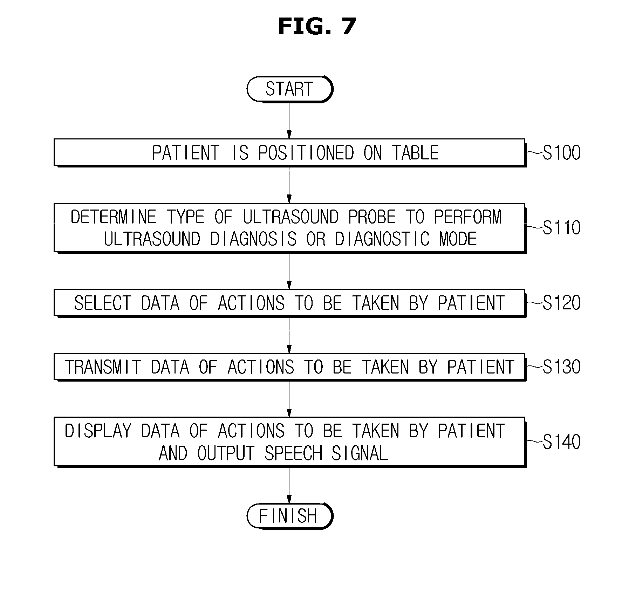

FIG. 7 is a flow chart illustrating a control method of the ultrasound diagnostic apparatus provided with a processor as to select data of actions to be taken by a patient in accordance with one embodiment of the present disclosure.

As illustrated on FIG. 2, FIG. 3, and FIG. 4, a patient needed to receive an ultrasound diagnosis may be positioned on a diagnostic table or a diagnostic bed (S100). The patient is needed to take proper actions as to receive the diagnosis on the table or the bed according to the portion of the ultrasound diagnosis or the type of the diagnosis.

The probe-type determining unit 230 may be able to determine the type of the ultrasound probe provided to perform an ultrasound diagnosis according to the control of the processor 200, and the processor 200 may be able to determine the mode of the ultrasound diagnosis (S110). As illustrated on FIG. 5, the types and the shapes of the ultrasound probes may be different to each other according to the portion and purpose of ultrasound diagnosis. Depending on the type of the ultrasound probe, the male connector 140 and the female connector 145 corresponding to the male connector 140 are varied, and thus the probe-type determining unit 230 may be able to determine the type of the ultrasound probe on the basis of the pre-stored information with respect to the ultrasonic probes according to the portions of diagnosis, when the male connector 140 is connected to the female connector 145. The probe-type determining unit 230 may be able to determine the type of the ultrasound probe, and may be able to transmit the determined data to the processor 200.

As described on FIG. 6, the processor 200 may be able to determine the mode of ultrasound diagnosis, and may be able to select the data of actions to be taken by a patient on the basis of the determined mode of ultrasound diagnosis from the stored data of actions of the patient. The mode of ultrasound diagnosis may include the modes with respect to portions of diagnosis, types of diagnosis, or ultrasound images. As described on FIG. 2, FIG. 3 and FIG. 4, the portions of diagnosis may be corresponded to the portions, such as a heart, an abdomen, and a carotid, that are needed to be diagnosed with respect to a subject. When the portions of ultrasound diagnosis are determined, based on the determined portions of ultrasound diagnosis, the data of actions to be taken by a patient may be selected from the data of actions of the patient. The types of ultrasound diagnosis as well may be changed according to the portions of diagnosis. As described on FIG. 1, as for the modes related to the ultrasound images, the A-mode (Amplitude mode), the B-mode (Brightness mode), the D-mode (Doppler mode), the E-mode (Elastography mode), and the M-mode (Motion mode) may be presented as examples.

The processor 200 may be able to select the data of actions to be taken by a patient on the basis of the data transmitted from the probe-type determining unit 230 or the determined data of the modes of ultrasound diagnosis (S120). On the basis of the determined type of the probe or the mode of ultrasound diagnosis, the data of actions to be taken by a patient may be selected from the data of actions of the patient according to the portions of ultrasound diagnosis, which are pre-stored at the storage unit 240. As described on FIG. 2, FIG. 3 and FIG. 4, the data of actions of the patient according to the portions of ultrasound diagnosis are referred to as the actions to be taken by the patient provided with the diagnosis, and thus overlapping descriptions will be omitted.

The data of actions of the patients selected by use of the processor 200 may be transmitted to the first display unit 160 and the second display unit 163, and may also be transmitted to the first alarm unit 170 and the second alarm unit 164.

The first display unit 160 may be installed at the body 105 of the ultrasound diagnostic apparatus, and thus the data of actions of a patient may be transmitted to the first display unit 160 by use of wire.

The second display unit 163 is separated from the ultrasound diagnostic apparatus 100 and may be installed at a position so that a patient may be able to check a screen in a state of lying down on a bed or a diagnostic table for an ultrasound diagnosis, and at this time, without going through the communication unit 220, the data may be received by use of wire, or the data may be wirelessly received through the communication unit 220. The communication unit 220 may be able to wirelessly transmit the data received from the processor 200 to the second display unit 163.

The first alarm unit 170 may be installed at the body 105 of the ultrasound diagnostic apparatus, and thus the processor 200 may be able to transmit the data of actions of a patient to the first alarm unit 170 by use of wire.

The second alarm unit 164 may be positioned at one side surface of the second display unit 163, and may be of an alarm unit in the form of a speaker that may be recognized from an outside, or may be an alarm unit in the form of a speaker provided at the second display unit 163. The second alarm unit 164 may be able to receive data by use of wire without going through the communication unit 220, and may be able to wirelessly receive data through the communication unit 220. The communication unit 220 may be able to wirelessly transmit the data received from the processor 200 to the second alarm unit 164.

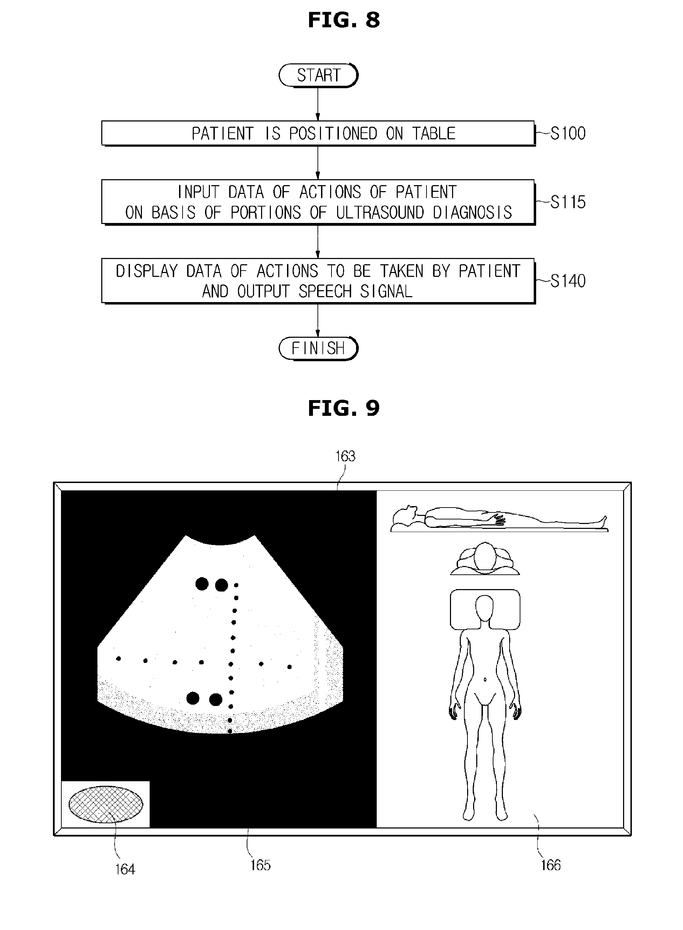

The first display unit 160 and the second display unit 163 may be able to display data of actions to be taken by a patient, and the first alarm unit 170 and the second alarm unit 164 may be able to output the data of actions to be taken by a patient in the form of speech signal (S140). Hereinafter, the screen configured to display the data of actions will be described by referring to FIG. 9 to FIG. 13.

FIG. 9 is a drawing illustrating the second display unit at which the data of actions to be taken by a patient is displayed at the time of performing an ultrasound diagnosis on an abdomen in accordance with one embodiment of the present disclosure.

As illustrated on FIG. 9, the second display unit 163 may include the second alarm unit 164, and may be able to simultaneously display ultrasound diagnostic images 165 with respect to a patient and actions to be taken 166 by the patient on a screen. On FIG. 9, the actions to be taken 166 by the patient as a whole is referred to as an erect posture while lying down for a diagnosis of an abdomen, and thus the posture may be displayed at a domain on a right side of the second display unit 163, and the patient may be able to make a posture for the diagnosis of the abdomen while referring to the posture displayed on the screen. As illustrated, the actions to be take by the patient may be displayed as an image seen from the above the patient as the patient is provided to lie down, an image from a side seen from the side of the head of the patient, and an image seen from a side of the body of the patient, while no limitation as to the direction or the angle is given. On FIG. 9, by dividing the domain of the screen of the second display unit, the ultrasound diagnostic images are displayed on the left side of the screen and the actions to be taken by the patient are displayed on the right side of the screen, while the dispositions of the screen are not limited, and a certain one of the ultrasound diagnostic images or the actions to be taken by the patient may be displayed on the screen. In addition, two different screens may be overlapped with respect to each other, and the screen may be disposed in various shapes.

The second alarm unit 164 included in the second display unit 163 may be able to output the data of actions to be taken by a patient at the time of an ultrasound diagnosis in the form of speech signal so that the patient or a test operator may be able to recognize, and the speech signal may be of a signal in the form of human voice providing detailed descriptions of the actions that the patient is needed to take, or may be of a mechanical sound as to simply have the patient recognize the data of actions or the data of actions displayed on a screen. In a case of the mechanical sound, when the actions to be taken by the patient is displayed on the screen, a mechanical sound, such as "Beep," may be output so that the patient may be able to recognize the actions to be taken by referring to the screen.

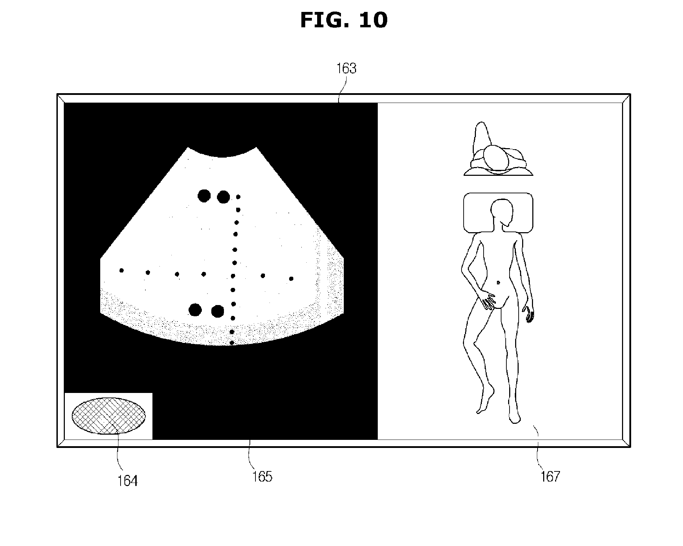

FIG. 10 is a drawing illustrating the second display unit at which the data of actions to be taken by a patient is displayed at the time of performing an ultrasound diagnosis on a carotid in accordance with one embodiment of the present disclosure.

As similar as FIG. 9, on FIG. 10, the display ultrasound diagnostic images 165 with respect to a patient and actions to be taken 167 by the patient may be simultaneously displayed on a screen. On FIG. 10, the actions to be taken 167 by the patient as a whole is referred to as a posture of comfortably lying down while the back of the patient is placed on a bed or a table for a diagnosis of a carotid as the face of the patient is turned toward a left side or a right side, and thus the posture may be displayed at a domain on a right side of the second display unit 163, and the patient may be able to make a posture for the diagnosis of the carotid while referring to the posture displayed on the screen. The detailed descriptions with respect to the displays on the screen of the second display unit 163 and the descriptions with respect to the output of an alarm of the second alarm unit 164 are already described on FIG. 9, and thus the overlapping descriptions will be omitted.

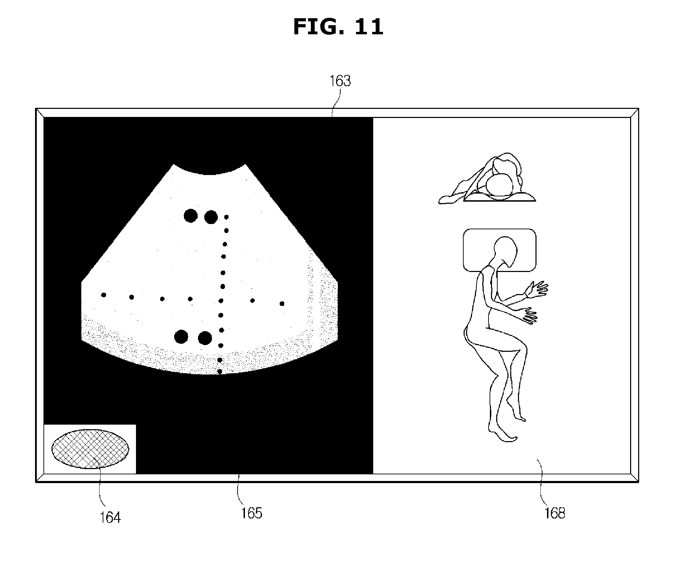

FIG. 11 is a drawing illustrating the second display unit at which the data of actions to be taken by a patient is displayed at the time of performing an ultrasound diagnosis on a heart in accordance with one embodiment of the present disclosure.

As similar as FIG. 9, on FIG. 11, the display ultrasound diagnostic images 165 with respect to a patient and actions to be taken 168 by the patient may be simultaneously displayed on a screen. On FIG. 11, the actions to be taken 168 by the patient as a whole is referred to as a posture of lying down toward a left side in a reclined manner while the right arm is placed on the right side of the body of the patient or comfortably positioned as the left arm is stretched toward upper side and the legs are bent for a diagnosis of a heart, and thus the posture may be displayed at a domain on a right side of the second display unit 163, and the patient may be able to make a posture for the diagnosis of the heart while referring to the posture displayed on the screen. The detailed descriptions with respect to the displays on the screen of the second display unit 163 and the descriptions with respect to the output of an alarm of the second alarm unit 164 are already described on FIG. 9, and thus the overlapping descriptions will be omitted.

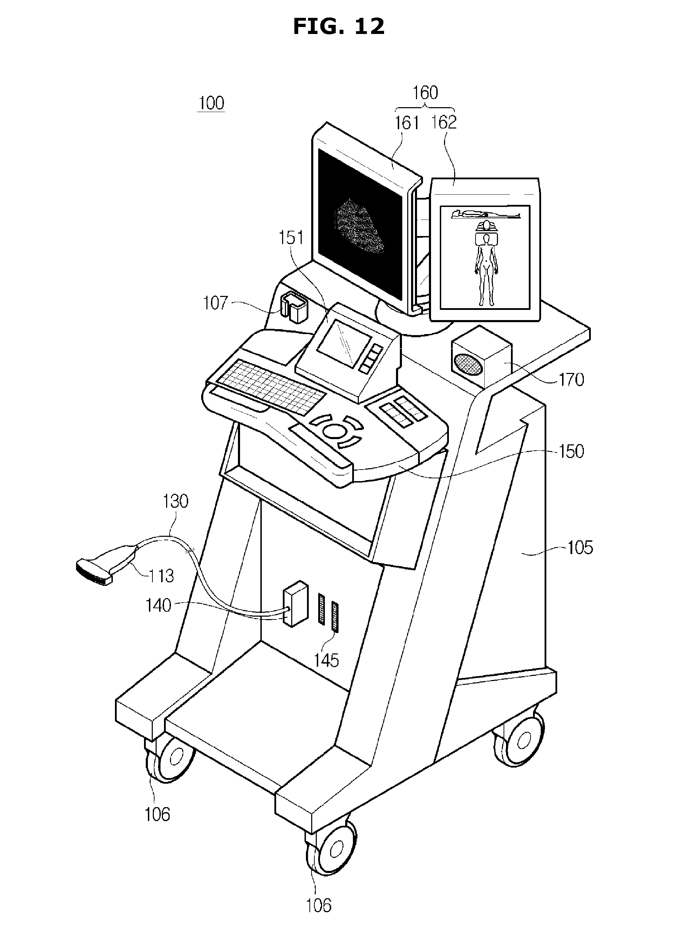

FIG. 12 is a perspective view illustrating the ultrasound diagnostic apparatus having a first display unit into which an ultrasound diagnostic apparatus for an abdomen is coupled at the time of an ultrasound diagnosis of an abdomen and at which the data of actions to be taken by a patient is displayed in accordance with one embodiment of the present disclosure.

As illustrated on FIG. 12, the ultrasound probe for an abdomen 113 may be coupled to the ultrasound diagnostic apparatus 100 as to perform an ultrasound diagnosis with respect to an abdomen. When the male connector of the ultrasound probe for an abdomen 113 is coupled to the female connector, the probe-type determining unit 230 is provided to determine the type of the coupled ultrasound probe and then transmit the data with respect to the type of the coupled ultrasound probe to the processor 200, and the processor 200, after selecting the data of actions to be taken by a patient from the determined type of the ultrasound probe, is provided to transmit the selected data of actions to the first display unit 160 and the first alarm unit 170. The first display unit may be divided into the first display apparatus 161 and the second display apparatus 162. The ultrasound diagnostic images may be displayed at the first display apparatus 161, and the actions to be taken by a patient may be displayed at the second display apparatus 162. In addition, the ultrasound diagnostic images and the actions to be taken by the patient may be simultaneously displayed at the each of the first display apparatus 161 or the second display apparatus 162. The ultrasound diagnostic images are output at the first display apparatus 161 by use of the first display unit 160 of FIG. 12, and the actions to be taken by the patient for the diagnosis of an abdomen are output at the second display apparatus 162 also by use of the first display unit 160 of FIG. 12. A test operator or a user, while referring to the screen being output at the first display unit 160, may be able to check if the ultrasound diagnostic images and the actions to be taken by the patient are properly output, and in a case when an error is present with respect to the output, an input may be performed through the input unit 150 as to change or reset the date of actions to be taken by the patient. The patient as well may be able to check the ultrasound diagnostic images and the actions to be taken being output at the first display unit 160.

The first alarm unit 170 mounted at the body 105 may be able to output the data of actions to be taken by a patient in the form of speech signal so that the patient or a test operator may be able to recognize. From the speech signal being output, the both the test operator and the patient may be able to check if the actions for the ultrasound diagnosis are properly taken.

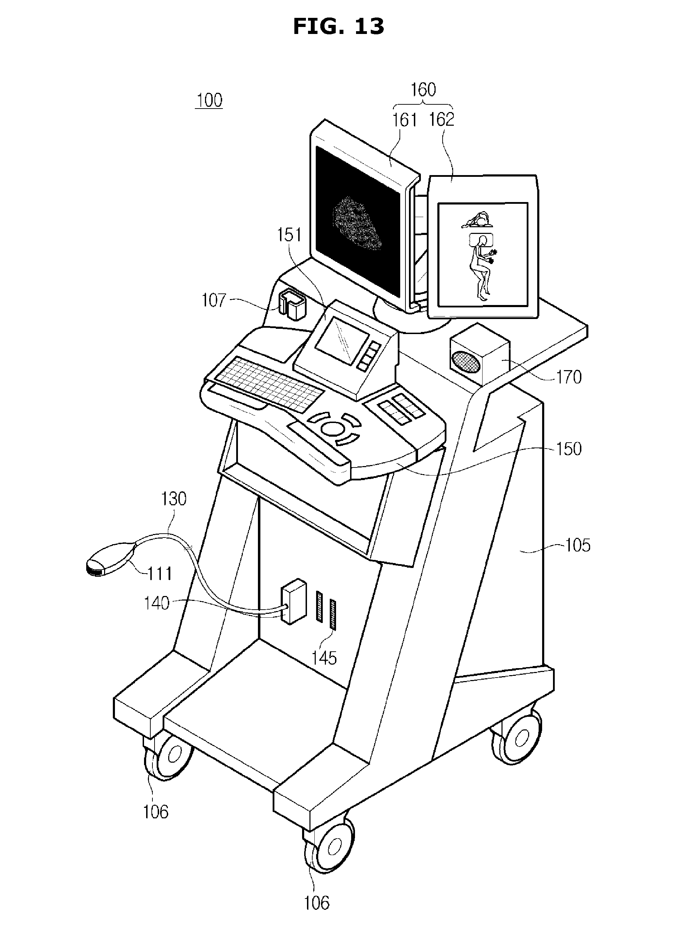

FIG. 13 is a perspective view illustrating the ultrasound diagnostic apparatus having the first display unit into which an ultrasound diagnostic apparatus for a heart is coupled at the time of an ultrasound diagnosis of a heart and at which the data of actions to be taken by a patient is displayed in accordance with one embodiment of the present disclosure.

As illustrated on FIG. 13, the ultrasound probe for a heart 111 may be coupled to the ultrasound diagnostic apparatus 100 as to perform an ultrasound diagnosis with respect to a heart. When the male connector of the ultrasound probe for a heart 111 is coupled to the female connector, the probe-type determining unit 230 is provided to determine the type of the coupled ultrasound probe and then transmit the data with respect to the type of the coupled ultrasound probe to the processor 200, and the processor 200, after selecting the data of actions to be taken by a patient from the determined type of the ultrasound probe, is provided to transmit the selected data of actions to the first display unit 160 and the first alarm unit 170. The ultrasound diagnostic images are output at the first display apparatus 161 by use of the first display unit 160 of FIG. 13, and the actions to be taken by the patient for the diagnosis of an abdomen are output at the second display apparatus 162 also by use of the first display unit 160 of FIG. 13.

The first alarm unit 170 mounted at the body 105 may be able to output the data of actions to be taken by a patient in the form of speech signal so that the patient or a test operator may be able to recognize.

Referring back to FIG. 7, the data of actions to be taken by a patient may be displayed and speech signals may be output through the first display unit 160, the second display unit 163, the first alarm unit 170, and the second alarm unit 164, and the patient may be able to precisely take the actions for an ultrasound diagnosis by use of the screen displayed at the display units and the speech signal being heard from the alarm units.

FIG. 8 is a flow chart illustrating a method of controlling the ultrasound diagnostic apparatus provided as to input the data of actions to be taken by a patient through an input unit in accordance with one embodiment of the present disclosure.

As similar to FIG. 7, as illustrated on FIG. 2, FIG. 3, and FIG. 4, a patient needed to receive an ultrasound diagnosis may be positioned on a diagnostic table or a diagnostic bed (S100). The patient is needed to take proper actions as to receive the diagnosis on the table or the bed according to the portion of the ultrasound diagnosis or the type of the diagnosis.

A test operator or a user may be able to input the data of actions of a patient through the input unit 150 on the basis of the portions of ultrasound diagnosis (S115). The data of actions to be taken by the patient may be selected from the data of actions of the patient, which are pre-stored at the storage unit 240, according to the portions of ultrasound diagnosis, and the selected data of actions may be input. For example, in a case of performing an ultrasound diagnosis with respect to an abdomen, the data of actions to be taken by a patient may be input through the input unit 150 at the time of performing the diagnosis of the abdomen, and in a case of performing an ultrasound diagnosis with respect to a heart, the data of actions to be taken by a patient may be input through the input unit 150 at the time of performing the diagnosis of the heart.

The first display unit 160 and the second display unit 163 may be able to display data of actions to be taken by a patient, and the first alarm unit 170 and the second alarm unit 164 may be able to output the data of actions to be taken by a patient in the form of speech signal (S140). The descriptions that are overlapped with respect to the descriptions provided on FIG. 7 will be omitted.

As information about actions to be taken by a patient at the time of performing an ultrasound diagnosis is provided by use of a display or audio-visually, the patient can easily take actions, and thus convenience of an ultrasound scan can be enhanced. An operator performing the ultrasound diagnosis can also easily scan without having to verbally and continually inform the patient of the actions to be taken or maintained by the patient at the time of performing the ultrasound scan.

Although a few embodiments of the present disclosure have been shown and described, it would be appreciated by those skilled in the art that changes may be made in these embodiments without departing from the principles and spirit of the disclosure, the scope of which is defined in the claims and their equivalents.

## Claims

1. An ultrasound diagnostic apparatus, comprising:
a storage unit at which data of actions of a patient needed to be taken at the time of an ultrasound diagnosis is stored;
a processor to determine a type of an ultrasound probe or a diagnostic mode provided to perform an ultrasound diagnosis and select a data of action to be taken by the patient from the stored data of actions on the basis of the determined type of the ultrasound probe or the determined diagnostic mode; and
at least one display unit to display the selected data of action to be taken by the patient.

2. The ultrasound diagnostic apparatus of claim 1, wherein:
the at least one display unit comprises a first display unit and a second display unit.

3. The ultrasound diagnostic apparatus of claim 1, comprising:
a first alarm unit to inform the selected data of action to be taken by the patient by use of an speech signal.

4. The ultrasound diagnostic apparatus of claim 2, wherein:
the second display unit comprises a second alarm unit to inform the selected data of action to be taken by the patient by use of an speech signal.

5. The ultrasound diagnostic apparatus of claim 4, comprising:
a communication unit to transmit the selected data of actions to be taken by the patient to the second display unit and the second alarm unit.

6. The ultrasound diagnostic apparatus of claim 1, comprising:
an input unit to receive an input of the data of actions to be taken by the patient.

7. The ultrasound diagnostic apparatus of claim 6, wherein:
the input unit receives an input as to change or reset the selected data of actions to be taken by the patient.

8. The ultrasound diagnostic apparatus of claim 1, further comprising:
a probe-type determining unit to determine the type of an ultrasound probe to perform the ultrasound diagnosis by recognizing the type of a male connector coupled to a female connector.

9. The ultrasound diagnostic apparatus of claim 2, wherein:
the first display unit and the second display unit simultaneously display an ultrasound diagnostic image and the selected data of actions to be taken by the patient.

10. An ultrasound diagnostic method, comprising:
determining a type of an ultrasound probe or a diagnostic mode provided to perform an ultrasound diagnosis;
selecting a data of action to be taken by a patient from stored data of actions on the basis of the determined type of the ultrasound probe or the determined diagnostic mode; and
displaying the selected data of action to be taken by the patient.

11. The ultrasound diagnostic method of claim 10, further comprising:
informing the selected data of action to be taken by the patient by use of an speech signal.

12. The ultrasound diagnostic method of claim 10, further comprising transmitting the selected data of actions to be taken by the patient when the data of actions are selected.

13. The ultrasound diagnostic method of claim 10, comprising:
receiving an input of the data of actions to be taken by the patient.

14. The ultrasound diagnostic method of claim 10, wherein:
the determining of the type of an ultrasound probe comprises determining the type of the ultrasound probe to perform the ultrasound diagnosis by recognizing the type of a male connector coupled to a female connector.

15. The ultrasound diagnostic method of claim 10, wherein:
the displaying of the selected data of actions to be taken by the patient comprises simultaneously displaying an ultrasound diagnostic image and the selected data of actions to be taken by the patient.
